Europäisches Patentamt

European Patent Office

Office européen des brevets

(19)

(11) Veröffentlichungsnummer: **0 010 321**
**A1**

(12) # EUROPÄISCHE PATENTANMELDUNG

(21) Anmeldenummer: 79200551.4

(22) Anmeldetag: 01.10.79

(51) Int. Cl.³: **A 61 B 10/00**

(30) Priorität: 19.10.78 CH 10828/78

(43) Veröffentlichungstag der Anmeldung:
30.04.80 Patentblatt 80/9

(84) Benannte Vertragsstaaten:
BE DE FR GB IT NL SE

(71) Anmelder: Brun Del Re, Renzo, Dr.
Kreuzackerweg 37
CH-4103 Bottmingen(CH)

(71) Anmelder: Schiele, Gerhard
Binningerstrasse 78
CH-4123 Allschwil(CH)

(72) Erfinder: Brun Del Re, Renzo,r.
Kreuzackerweg 37
CH-4103 Bottmingen(CH)

(72) Erfinder: Schiele, Gerhard
Binningerstrasse 78
CH-4123 Allschwil(CH)

(74) Vertreter: Eschmann, Heinz et al,
Holbeinstrasse 36-38
CH-4051 Basel(CH)

(54) Vorrichtung zur einhändigen Bedienung eines Biopsiegerätes.

(57) Zur Bedienung eines Biopsiegerätes (1, 2, 3, 4) benötigt der Arzt normalerweise beide Hände. Die Vorrichtung zur einhändigen Bedienung des Geräts weist eine verschiebbar gelagerte Hülse (6) auf, die unter dem Einfluss einer Feder (10) steht und durch einen Auslösemechanismus (15) freigegeben und somit durch die Feder (10) nach vorne über die Einbuchtung (1a) des Mandrins (1) gestossen wird. Damit hat der Arzt eine Hand frei, um beispielsweise das zu untersuchende Gewebe festzuhalten.

EP 0 010 321 A1

./...

Croydon Printing Company Ltd.

FIG.1

Die Erfindung betrifft eine Vorrichtung zur einhändigen Bedienung eines Biopsiegerätes zwecks Entnahme einer Gewebeprobe aus dem menschlichen oder tierischen Körper, wobei das Biopsiegerät einen eine Spitze aufweisenden Mandrin umfasst, welcher zur Aufnahme der Gewebeprobe eine Einbuchtung aufweist und in einer eine Schneidkante besitzenden Hohlnadel zwischen einer hinteren und einer vorderen Endlage gleitend gelagert ist.

Das Prinzip des Biopsiegerätes ist bekannt. Es besteht darin, dass eine Hohlnadel mit Mandrin, der hinter der scharfen Schneide eine 2 cm lange Aussparung aufweist, ins Gewebe eingeführt wird. Der Mandrin wird sodann vorgeschoben. Das Gewebe, das die Aussparungen ausfüllt, wird durch die schnell vorgestossene Hohlnadel vom übrigen Gewebe abgetrennt.

Das Biopsiegerät zum Einmalgebrauch bewährt sich bei unverschieblichem Gewebe, bei zweihändiger Bedienung. Bei verschieblichem Gewebe muss jedoch zusätzlich dieses durch eine Zweitperson festgehalten werden. Vor allem wenn es sich z.B. um einen derberen Gewebeknoten in der Brust handelt, kann dieser, wenn er nicht fixiert wird, von der Nadel abgedrängt werden, so dass schliesslich die Biopsie ausserhalb des zu untersuchenden Knotens erfolgt, was zu einer Fehldiagnose führen kann. Um einen möglichst grossen Gewebezylinder gewinnen zu können, muss zudem die Hande, die den Mandrin führt, beim Vorschieben der Hohlnadel absolut ruhig gehalten werden. Wie zahlreiche Versuche gezeigt haben, haben die meisten Operateure die Tendenz, bei dieser Schneidebewegung beide Hände zu bewegen, d.h., sowohl die Hohlnadel vorzuschieben, wie gleichzeitig den Mandrin zurückzuziehen. Dadurch wird jedoch das Gewebe aus der Aussparung wieder herausgezogen und der gewonnene Gewebezylinder wird dadurch entsprechend kürzer und mechanisch beschädigt. Die Aussagekraft der histologischen Untersuchung dieses Zylinders ist dadurch wiederum weniger repräsentative oder unmöglich.

Es ist daher Aufgabe der Erfindung, unter Vermeidung der Nachteile des Bekannten eine Vorrichtung zur einhändigen Bedienung des Biopsiegerätes zu schaffen, welche konstruktiv einfach aufgebaut, sterilisierbar ist und keine Abänderung des handelsüblichen Biopsiegerätes erfordert.

Diese Aufgabe wird dank der vorliegenden Erfindurch eine Vorrichtung gelöst, welche die im Patentanspruch 1 definierte Merkmalskombination aufweist.

Auf der beiliegenden Zeichnung ist ein Ausführungsbeispiel des Erfindungsgegenstandes veranschaulicht.

Fig. 1 ist eine vereinfachte Schnittdarstellung dieser Ausführungsform mit eingesetztem Biopsiegerät,

Fig. 2 ist eine Rückansicht der Vorrichtung gemäss Fig. 1,

Fig. 3 zeigt die gleiche Vorrichtung im entspanntem Zustand.

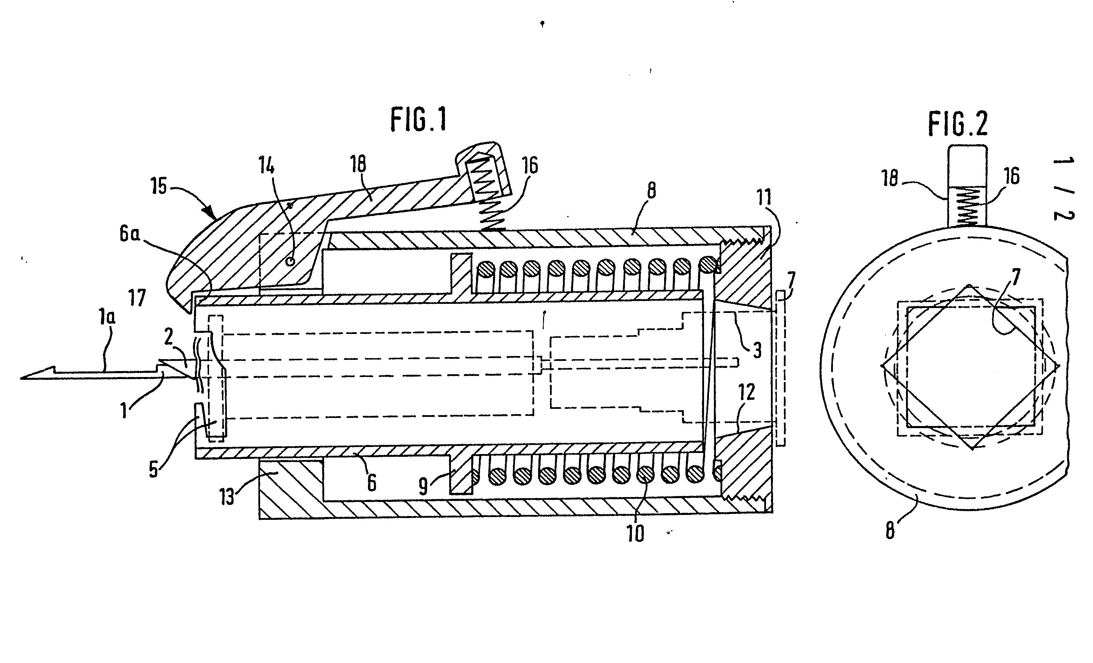

Das in Fig. 1 nur schematisch angedeutete Biopsiegerät weist einen Mandrin 1 auf, der zwischen zwei Endstellungen gleitbar in einer Hohlnadel 2 gelagert ist. Der Mandrin 1 ist in einem Mandrinhalter 3, die Hohlnadel 2 in einem Hohlnadelhalter 4 fest verankert. Der Hohlnadelhalter 4 ist mittels eines Bajonettverschlusses 5 im vorderen Endabschnitt einer Hülse 6 befestigt, während der Mandrinhalter 3 mit seinem Flansch 7 an der hinteren Stirnwand eines Gehäuses 8 anliegt. Der Mandrinhalter 3 kann mit dem Mandrin 1 bezüglich der Hohlnadel 2 hin- und herbewegt werden. Die vordere, in Fir. 1 angedeutete Einstechlage ist dadurch begrenzt, dass der Flansch 7 am Gehäuse 8 anstösst.

Die Hülse 6 ist im Gehäuse 8 beweglich angeordnet und weist etwa in ihrem Mittelbereich einen Ringflansch 9 auf, der einer Druckfeder 10 als Federteller dient. Das gegenüberliegende Ende der Druckfeder 10 stützt sich auf die ringförmige Innenfläche eines Gewindestopfens 11, der in das Gehäuse 8 eingeschraubt ist und eine zentrale Bohrung 12 für den Durchtritt des Mandrinhalters 3 besitzt.

Das Gehäuse 8 ist in seinem vorderen Ende mit einer nach innen weisenden Ringschulter 13 versehen, die den Verschiebeweg der Hülse 6 durch Anschlag am Ringflansch 9 begrenzt. An der Ringschulter 13 ist mittels einer Schwenkachse 14 ein insgesamt mit 15 bezeichneter Auslösemechanismus befestigt, der einen zweiarmigen Hebel 18 aufweist, am einen Ende unter dem Einfluss einer Druckfeder 16 steht und am anderen Ende eine Sperrnase 17 besitzt. Durch die Feder 16 wird der Auslösemechanismus 15 somit, wie Fig. 1 zeigt, in die Sperrstellung gedrückt, in welcher die Vorderkante 6a der Hülse 6 an der Sperrnase 17 anliegt und die Hülse somit in ihrer dargestellten Lage arretiert ist.

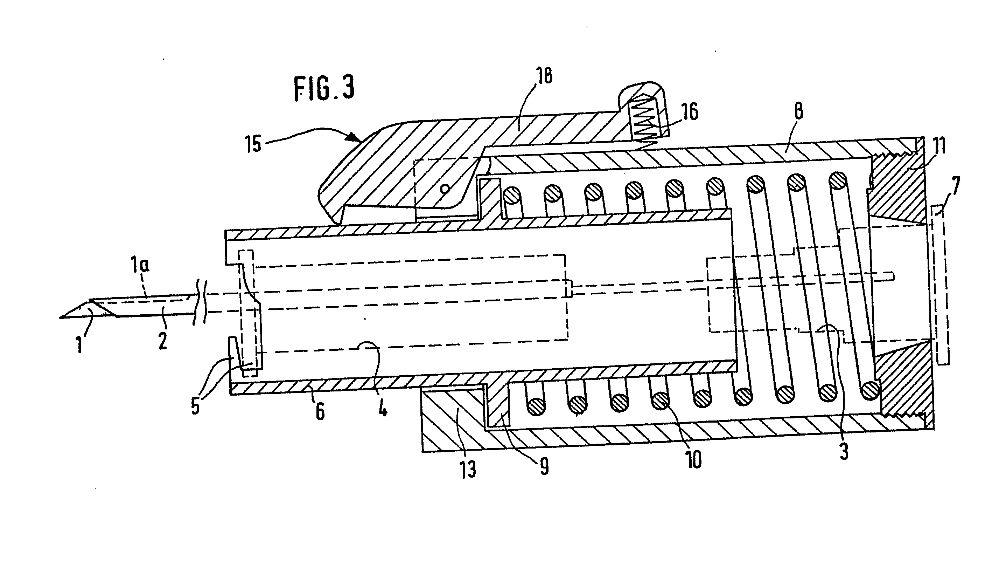

Zur Entnahme einer Gewebeprobe wird das Biopsiegerät, bestehend aus dem Mandrin 1 und der Hohlnadel 2, zunächst wie üblich bis zu dem untersuchenden Gewebeteil vorgestossen, worauf der Mandrin mit dem Daumen oder mit dem Hohlhand bis zum Anschlag des Flansches 7 am Gehäuse 8 weiter vorgeschoben wird. Nun wird durch Druck auf den Hebel 18 die Hülse 6 freigegeben, die unter dem Einfluss der Druckfeder 10 nach vorne schnellt und das in der Aussparung 1a (Fig. 1) befindliche Gewebe abtrennt und in dieser Aussparung einschliesst (Fig. 3). Anschliessend wird die Hohlnadel 2 mit dem Mandrin 1 zurückgezogen und die Gewebeprobe kann der Aussparung 1a nach dem Zurückschieben der Hohlnadel 2 entnommen werden.

Während des gesamten beschriebenen Vorganges hat der Arzt eine Hand frei und kann mit derselben beispielsweise die Spitze des Biopsiegerätes führen (z.B. bei der transrektalen Prostatabiopsie) oder das verschiebliche Gewebe festhalten (z.B. bei beweglichem Brusttumor).

Um ein unbeabsichtigtes, vorzeitiges Auslösen zu verhindern, kann der Auslösemechanismus 15 beispielsweise durch ein Sperrglied ergänzt werden. Derartige Sperrglieder sind dem Fachmann bekannt und brauchen daher nicht dargestellt bzw. beschrieben zu werden. Das Sperrglied hemmt jedenfalls die Auslösebewegung des Hebels 18, so dass eine Auslösung nur möglich ist, wenn das Sperrglied vor dem Hebel 18 bzw. gleichzeitig mit diesem betätigt wird.

Da das gesamte Biopsiegerät vorzugsweise von vorne in das Gehäuse 8 bzw. durch die Hülse 6 geschoben wird, kann der Flansch 7 quadratisch ausgebildet werden, so dass er zwar durch die Oeffnung 12 hindurchschiebbar ist, nach einer Teildrehung jedoch als Anschlag dient.

Die Figuren 1 bis 3 zeigen lediglich ein Ausführungsbeispiel, das vom Fachmann in mannigfaltiger Hinsicht im Rahmen des durch den Patentanspruch 1 umrissenen Schutzumfanges abgewandelt werden kann. So könnte der Auslösemechanismus 15 beispielsweise in Form eines Pistolengriffes ausgebildet werden.

Anstelle der Feder (10) liesse sich auch eine andere Antriebsvorrichtung verwenden, beispielsweise ein Hebelmechanismus, der von Hand bis zur Auslösung unter Vorspannung gehalten wird, eine pneumatisch oder elektromagnetisch betätigte Vorrichtung etc.

Auch kann es von Vorteil sein, wenn die Hohlnadel 2 beim Auslösen durch den Antriebsmechanismus, im vorliegenden Falle die Feder 10, in Rotation versetzt und damit die Scherwirkung verbessert wird.

- 6 -

Patentansprüche

1. Vorrichtung zur einhändigen Bedienung eines Biopsiegerätes zwecks Entnahme einer Gewebeprobe aus dem menschlichen oder tierischen Körper, wobei das Biopsiegerät einen eine Spitze aufweisenden Mandrin umfasst, welcher zur Aufnahme der Gewebeprobe eine Einbuchtung aufweist und in einer eine Schneidkante aufweisenden Hohlnadel zwischen einer hinteren und einer vorderen Endlage gleitend gelagert ist, gekennzeichnet durch ein Gehäuse (8), innerhalb dessen Mittel zur Lagerung der der Mandrinspitze abgewandten Abschnittes des Mandrins (1) und der Hohlnadel (2) angeordnet sind, wobei zur Lagerung der Hohlnadel (2) ein verschiebbar im Gehäuse (8) angeordnete Halteglied (9) dient, das einerseits unter dem Einfluss einer Antriebsvorrichtung (10) steht, die dasselbe in Richtung der Mandrinspitze zu drücken trachtet, andererseits mit einem Auslösemechanismus (15) gekoppelt ist, das Ganze derart, dass die Hohlnadel (2) bei Betätigung des Auslösermechanismus (15) eine rasche Relativbewegung bezüglich des in seiner vorderen Endlage befindlichen Mandrins (1) ausübt, dabei über die Einbuchtung (1a) des Mandrins (1) nach vorne schnellt und die in der Einbuchtung befindliche Gewebeprobe abtrennt.

2. Vorrichtung nach Anspruch 1, dadurch gekennzeichnet, dass die Hohlnadel (2) lösbar an einer Hülse (6) verankert ist, die ihrerseits innerhalb eines Griffgehäuses (8) verschiebbar gelagert ist, wobei die in Form einer Feder (10) ausgebildete Antriebsvorrichtung zwischen der hinteren Stirnwand (11) des Griffgehäuses (8) und einem am Umfang der Hülse (6) angeordneten Ringflansch (9) eingespannt ist.

3. Vorrichtung nach Anspruch 1, dadurch gekennzeichnet, dass der Auslösemechanismus (15) einen schwenkbar gelagerten Hebel (18) aufweist, dessen mit einer Sperrnase (17) versehener vorderer Abschnitt die Vorderkante der Hülse (6) in deren Verriegelungsstellung übergreift (Fig. 1).

4. Vorrichtung nach Anspruch 2, dadurch gekennzeichnet, dass der Mandrin (1) in einem Halter (3) verankert ist, der durch eine zentrale Oeffnung (12) des Griffgehäuses (8) hindurchragt, und dass der Halter (3) ferner an seinem freien Ende einen Anschlag (7) aufweist, der die Vorwärtsbewegung des Mandrins (1) im Zusammenwirken mit der Aussenfläche der Stirnwand des Gehäuses (8) begrenzt.

5. Vorrichtung nach einem der Ansprüche 1 bis 4, dadurch gekennzeichnet, dass der Auslösemechanismus (15) mindestens annähernd in Form eines Pistolengriffes ausgebildet ist.

6. Vorrichtung nach einem der Ansprüche 1 bis 5, dadurch gekennzeichnet, dass der Auslösemechanismus (15) mit einem Sicherungsorgan kombiniert ist, welches die Betätigung des Auslösemechanismus nur bei vorheriger oder gleichzeitiger Betätigung des Sicherungsorganes gestattet.

0010321

FIG.2

FIG.1

FIG.3

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - B - 1 160 573 (Dr. P. LUDWIG) <br> + Gesamt + <br> -- | 1,3,4,5 |
| | DE - B - 1 817 555 (BAXTER LAB.) <br> + Spalte 4, Zeile 2 - Spalte 5, Zeile 43; Figuren + <br> -- | 1,4 |
| | CH - A - 483 829 (Dr. H. REINISCH) <br> + Spalte 2, Zeile 5 - Spalte 3, Zeile 43; Figuren + <br> -- | 1-5 |
| | US - A - 3 902 498 (MINNESOTA MINING) <br> + Spalte 5, Zeile 27 - Spalte 6, Zeile 19; Spalte 7, Zeile 53 - Spalte 9, Zeile 2; Figuren 1 - 4 + <br> -- | 1,5 |
| A | US - A - 3 844 272 (A. BANKO) <br> + Spalte 3, Zeile 18 - Spalte 5, Zeile 43; Figuren 1 - 8 + <br> -- | |
| A | US - A - 3 561 429 (EVERSHARP INC.) <br> + Spalte 2, Zeilen 12 - 72; Figuren 1 - 6 + <br> -- | 5 |
| A | DE - A1 - 2 721 012 (BAYLIS SHELBY M.) <br> + Gesamt + <br> -- | |

### KLASSIFIKATION DER ANMELDUNG (Int. Cl.³)

A 61 B 10/00

### RECHERCHIERTE SACHGEBIETE (Int. Cl.³)

A 61 B 10/00
A 61 B 17/00
G 01 N 1/00

### KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| WIEN | 03-01-1980 | LUDWIG |

EPA form 1503.1   06.78

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| P | <u>DE - A1 - 2 719 959</u> (B. BRAUN MELSUNGEN AG)<br><br>+ Gesamt +<br><br>& FR-A1-2 389 365 (01-12-1978)<br><br>& SE-A -7 805 094 (05-11-1978)<br><br>-- | | |
| | | | RECHERCHIERTE SACHGEBIETE (Int. Cl.²) |

EPA Form 1503.2  06.78